(19) Europäisches Patentamt
European Patent Office
Office européen des brevets

(11) Numéro de publication : **0 395 546 B1**

(12) # FASCICULE DE BREVET EUROPEEN

(45) Date de publication du fascicule du brevet : **29.09.93 Bulletin 93/39**

(51) Int. Cl.[5] : **C07C 57/03,** C07C 51/12

(21) Numéro de dépôt : **90420204.1**

(22) Date de dépôt : **24.04.90**

(54) **Procédé de préparation d'acides beta,gamma-insaturés.**

(30) Priorité : **28.04.89 FR 8906019**

(43) Date de publication de la demande : **31.10.90 Bulletin 90/44**

(45) Mention de la délivrance du brevet : **29.09.93 Bulletin 93/39**

(84) Etats contractants désignés : **AT BE CH DE DK ES FR GB GR IT LI LU NL SE**

(56) Documents cités :
**DE-A- 3 345 375**
**US-A- 3 338 961**
**US-A- 3 437 676**

(73) Titulaire : **RHONE-POULENC CHIMIE**
**25, quai Paul Doumer**
**F-92408 Courbevoie Cédex (FR)**

(72) Inventeur : **Denis, Philippe**
**16 Allée des Troenes**
**F-69150 Decines (FR)**
Inventeur : **Jenck, Jean**
**1, Impasse du Rotagnier**
**F-69680 Chassieu (FR)**
Inventeur : **Perron, Robert**
**La Pécolière**
**F-69390 Charly (FR)**

(74) Mandataire : **Vignally, Noel et al**
**Rhône-Poulenc Chimie Direction de la Propriété Industrielle Centre de Recherches des Carrières B.P. 62**
**F-69192 Saint-Fons Cédex (FR)**

**EP 0 395 546 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation d'acides β,γ-insaturés. Elle a plus particulièrement pour objet un procédé de préparation d'acides β,γ-insaturés par carbonylation d'alcools allyliques.

Dans le brevet des Etats Unis d'Amérique n° 4,189,608 est décrit un procédé de préparation de l'acide butène-3 oïque par mise en contact de l'alcool allylique et de l'oxyde de carbone en présence d'un catalyseur à base de chlorure de palladium à une température de 50 à 300°C, sous pression élevée, la réaction étant conduite dans un acide carboxylique en $C_2$-$C_{10}$ substantiellement anhydre comme solvant liquide.

Or, l'acide carboxylique est susceptible de réagir sur l'alcool pour donner naissance à un carboxylate d'allyle qui deviendrait le réactif de la carbonylation catalysée par le palladium.

Par ailleurs, l'activité catalytique reste faible comme l'attestent les durées de réaction relativement longues.

Dans le brevet des Etats Unis d'Amérique n° 4,025,547 est décrite la carbonylation d'alcanols allyliques primaires en esters du type du vinylacétate d'allyle en présence d'un système catalytique homogène renfermant trois composants :

- un halogénure de palladium avec
- un ou plusieurs ligands donneurs du groupe VB et,
- un cocatalyseur qui est un halogènure d'un métal du groupe IVB.

Un exemple typique de ces compositions catalytiques est $PdCl_2(P(C_6H_5)_3)_2.SnCl_2$.

La triphénylphosphine comme le dichlorure d'étain peuvent être engagés en excès.

Dans ce procédé, on ne forme pas d'acide mais un ester allylique ; l'alcool allylique sert à la fois de substrat qui se carbonyle dans les conditions de la réaction et de réactif d'estérification. Par ailleurs, les pressions mises en oeuvre sont très élevées.

Dans la DOS 3 345 375 est décrit un procédé de carbonylation d'alcanols allyliques secondaires ou tertiaires en présence d'halogénures de palladium compléxés par une phosphine le cas échéant en excès, à 50-150°C sous pression élevée.

Si la sélectivité de la réaction semble appréciable, l'activité du système catalytique reste faible comme l'attestent à la fois les durées de réaction longues et les pressions très élevées qui sont mises en oeuvre dans ce procédé.

Il a maintenant été trouvé qu'il est possible d'obtenir des acides β,γ-insaturés par carbonylation d'alcools allyliques avec une efficacité améliorée notamment dans des conditions de pression plus douces que celles requises jusqu'alors.

La présente invention a donc pour objet un procédé de préparation d'acides β,γ-insaturés par mise en contact d'un alcool allylique, de l'oxyde de carbone et d'un catalyseur à base de palladium, à température élevée et sous une pression supérieure à la pression atmosphérique caractérisé en ce qu'on opère également en présence d'au moins un chlorure d'onium quaternaire d'un élément du groupe VB choisi parmi l'azote et le phosphore, ledit élément étant tétracoordonné à des atomes de carbone, l'azote pouvant être coordoné à deux atomes de phosphore pentavalents.

Dans le cadre du procédé selon la présente invention on entend par alcool allylique un composé mono ou dihydroxylé, insaturé répondant à la formule générale :

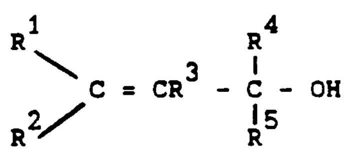

(I)

dans laquelle :

. $R^1$ à $R^5$ identiques ou différents représentent :
  - un atome d'hydrogène,
  - un radical alkyle, aryle ou aralkyle comportant au maximum 20 atomes de carbone,
  - un radical alcényle exempt d'insaturation terminale et renfermant au maximum 15 atomes de carbone,

. l'un quelconque des radicaux $R^1$ à $R^5$ peut en outre représenter un radical hydroxyméthyle ($-CH_2OH$),

. $R^1$(ou $R^2$) et $R^4$(ou $R^5$) peuvent en outre former ensemble un radical unique divalent alkylène comportant de 2 à 5 atomes de carbone.

Lorsque l'un des radicaux $R^1$ à $R^5$ représente un radical hydroxyméthyle, de préférence, l'ensemble des autres radicaux $R^1$ à $R^5$ représente un atome d'hydrogène.

Le groupe hydroxyméthyle est réactif dans les conditions du présent procédé et il a également été trouvé

de manière tout à fait surprenante qu'il est possible d'obtenir au départ du butène-2 diol-1,4 comme du butène-1 diol-3,4 ou de leurs mélanges de l'acide hexène-3 dioïque-1,6 avec une sélectivité appréciable. Or l'acide hexènedioïque-1,6 peut être hydrogéné en acide adipique.

L'acide adipique l'une des matières premières du nylon 66 est produit avec de forts tonnages et de ce seul fait, toute nouvelle voie d'accès à ce diacide et/ou à ses dérivés présente un intérêt de principe immédiatement perceptible.

Comme cela apparaît à la lecture de la formule générale (I) ci-avant sont susceptibles d'être mis en oeuvre dans le cadre du présent procédé des alcools allyliques primaires, secondaires ou tertiaires.

A titre d'exemples de tels alcools on peut citer :

- l'alcool allylique
- le butène-2 ol-1 (alcool crotylique)
- le butène-3 ol-2 (alcool méthyl-1 allylique)
- le pentène-1 ol-3
- l'hexène-1 ol-3
- l'octène-1 ol-3
- le diméthyl-3,7 octadiène-2,6 ol-1 (géraniol)
- le diméthyl-3,7 octadiène-1,6 ol-3 (linalol)
- le phényl-3 propène-2 ol-1 (alcool cinnamique)
- le méthyl-2 propène-1 ol-3
- le cyclohexène-2 ol-1
- le méthyl-3 butène-1 ol-3
- le butène-2 diol-1,4
- le butène-1 diol-3,4

Le procédé selon la présente invention est conduit en présence d'un catalyseur à base de palladium.

Bien que la nature précise de l'espèce (ou des espèces) catalytiquement active(s) dans la réaction en cause ne soit pas totalement élucidée, la Demanderesse a constaté que divers composés du palladium et le palladium métallique sont susceptibles d'être des précurseurs utiles dans la mise en oeuvre du présent procédé.

Parmi les sources de palladium pouvant être mises en oeuvre pour réaliser le procédé faisant l'objet de l'invention, on peut citer :

. le palladium métallique déposé le cas échéant sur un support, tel que le charbon, l'alumine, ou la silice,
. $PdCl_2$, $Pd(OAc)_2$, $PBU_4PdCl_3$(Bu = n-butyle)
. les sels ou les complexes $\pi$-allyliques de palladium, dont l'anion coordonné au cation Pd est choisi parmi les anions suivants : carboxylates tels que formiate, acétate, propionate, benzoate ; acétylacétonate, halogénures tels que $Cl^-$ et $Br^-$ et de préférence $Cl^-$ ;

La quantité précise de catalyseur à mettre en oeuvre, qui peut varier dans de larges limites, dépendra avant tout d'un compromis entre l'efficacité souhaitée et la dépense en catalyseur et des autres conditions choisies pour la réaction.

En général, de bons résultats peuvent être obtenus avec une concentration de palladium dans le milieu réactionnel comprise entre $10^{-3}$ et 1 mol/l. De préférence, cette concentration est comprise entre $2.10^{-3}$ et $5.10^{-2}$ Mol/l.

L'une des caractéristiques essentielles du présent procédé réside dans le fait que la réaction est conduite également en présence d'un chlorure d'onium quaternaire d'un élément du groupe VB choisi parmi l'azote et le phosphore, ledit élément étant tétracoordonné à des atomes de carbone, l'azote pouvant en outre être coordonné à deux atomes de phosphore pentavalents.

Par cation onium quaternaire dont l'élément du groupe VB est tétracoordonné à des atomes de carbone, on entend des cations formés à partir d'azote ou de phosphore et de quatre groupements hydrocarbonés monovalents, identiques ou différents, dont la valence libre est portée par un atome de carbone, chaque groupement étant relié à l'élément précité par ladite valence libre, deux quelconques de ces groupements pouvant par ailleurs former ensemble un radical divalent.

Pour une bonne réalisation du procédé de l'invention, le chlorure d'onium quaternaire présente un cation onium quaternaire répondant à l'une des formules II à V ci-après :

II) 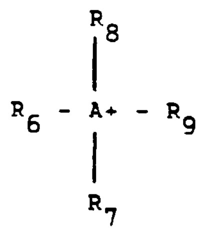

III) 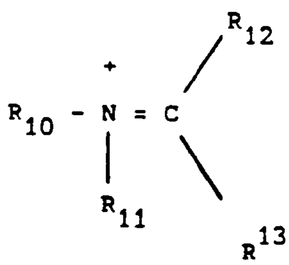

IV) 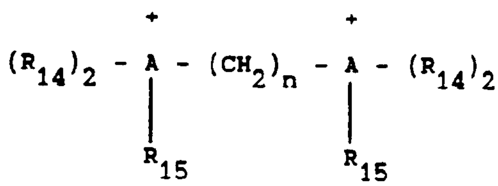

v) $(R_{16})_3 - P = N^+ = P - (R_{16})_3$

dans lesquelles :

- A représente de l'azote ou du phosphore
- $R_6$, $R_7$,$R_6$, $R_9$ sont identiques ou différents et réprésentent :
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préférence de 4 à 8 atomes de carbone,
  . un radical aryle contenant de 6 à 10 atomes de carbone, éventuellement substitué par un ou des radicaux alkyles contenant de 1 à 4 atomes de carbone, alcoxy, alcoxycarbonyle ou halogéno ;
  . deux desdits radicaux $R_6$ à $R_9$ pouvant former ensemble un radical alkylène, alcénylène ou alcadiènylène, linéaire ou ramifié et contenant de 3 à 6 atomes de carbone ;
- $R_{10}$, $R_{11}$, $R_{12}$, $R_{13}$ sont identiques ou différents et représentent :
  . un radical alkyle linéaire ou ramifié contenant de 1 à 4 atomes de carbone ;
  . les radicaux $R_{12}$ et $R_{13}$ pouvant former ensemble un radical alkylène, contenant de 3 à 6 atomes de carbone ;
  . les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec N un hétérocycle azoté ;
- $R_{14}$ représente un radical alkyle linéaire ou ramifié contenant de 1 à 4 atomes de carbone ou un radical phényle ;
- $R_{15}$ représente :
  . un radical alkyle linéaire ou ramifié contenant de 1 à 4 atomes de carbone, semblable ou différent de $R_{14}$ ;
  . un radical alcényle linéaire ou ramifié, contenant de 2 à 12 atomes de carbone, de préférence de 4 à 8 atomes de carbone ;
- n représente un nombre entier supérieur ou égal à 1 et inférieur ou égal à 10 et de préférence inférieur

ou égal à 6 ;

- $R_{16}$ représente un radical aryle contenant de 6 à 10 atomes de carbone, éventuellement substitué par un ou des groupes alkyle contenant de 1 à 4 atomes de carbone, alcoxy, alcoxycarbonyle ou halogéno.

A titre d'exemple de cations onium quaternaire répondant à la formule II on peut citer les cations :

. tétraméthylammonium,
. triéthylméthylammonium
. tributylméthylammonium
. triméthyl(n-propyl)ammonium
. tétraéthylammonium
. tétrabutylammonium
. dodécyltriméthylammonium
. méthyltrioctylammonium
. heptyltributylammonium
. tétrapropylammonium
. tétrapentylammonium
. tétrahexylammonium
. tétraheptylammonium
. tétraoctylammmonium
. tétradécylammonium
. butyltripropylammonium
. méthyltributylammonium
. pentyltributylammonium
. méthyldiéthylpropylammonium
. éthyldiméthylpropylammonium
. tétradodécylammonium
. tétraoctadécylammonium
. hexadécyltriméthylammonium
. benzyltriméthylammonium
. benzyldiméthylpropylammonium
. benzyldiméthyloctylammonium
. benzyltributylammonium
. benzyltriéthylammonium
. phényltriméthylammonium
. benzyldiméthyltétradécylammonium
. benzyldiméthylhexadécylammonium
. diméthyldiphénylammonium
. méthyltriphénylammonium
. butène-2 yltriéthylammonium
. N,N-diméthyl-tétraméthylènammonium
. N,N-diéthyl-tétraméthylènammonium
. tétraméthylphosphonium
. tétrabutylphosphonium
. éthyltriméthylphosphonium
. triméthylpentylphosphonium
. octyltriméthylphosphonium
. dodécyltriméthylphosphonium
. triméthylphénylphosphonium
. diéthyldiméthylphosphonium
. dicyclohexyldiméthylphosphonium
. diméthyldiphénylphosponium
. cyclohexyltriméthylphosphonium
. triéthylméthylphosphonium
. méthyl-tri(isopropyl)phosphonium
. méthyl-tri(n-propyl)phosphonium
. méthyl-tri(n-butyl)phosphonium
. méthyl-tri(méthyl-2 propyl)phosphonium
. méthyltricyclohexylphosphonium
. méthyltriphénylphosphonium

. méthyltribenzylphosphonium
. méthyl-tri(méthyl-4 phényl)phosphonium
. méthyltrixylylphosphonium
. diéthylméthylphénylphosphonium
. dibenzylméthylphénylphosphonium
. éthyltriphénylphosphonium
. tétraéthylphosphonium
. éthyl-tri(n-propyl)phosphonium
. triéthylpentylphosphonium
. hexadécyltributylphosphonium
. éthyltriphénylphosphonium
. n-butyl-tri(n-propyl)phosphonium
. butyltriphénylphosphonium
. benzyltriphénylphosphonium
. (β-phényléthyl)diméthylphénylphosphonium
. tétraphénylphosphonium
. triphényl(méthyl-4 phényl)phosphonium
. tétrakis(hydroxyméthyl)phosphonium
. tétrakis(hydroxy-2 éthyl)phosphonium

Parmi les cations répondant à la formule III, on peut citer les cations :
. N-méthylpyridinium
. N-éthylpyridinium
. N-hexadécylpyridinium
. N-méthylpicolinium

Parmi les cations répondant à la formule IV, on peut citer les cations :
. bis(triméthylammonium)-1,2 éthane
. bis(triméthylammonium)-1,3 propane
. bis(triméthylammonium)-1,4 butane
. bis(triméthylammonium)-1,3 butane

Parmi les cations répondant à la formule V, on peut citer les cations :
bis(triphénylphosphine)iminium
bis(tritolylphosphine)iminium.

On recourt avantageusement à ceux des cations onium répondant à la formule (II) ci-avant dans laquelle :
- A représente du phosphore et,
- $R_6$, $R_7$, $R_6$ et $R_9$ sont identiques ou différents et représentent un radical alkyle linéaire ou ramifié contenant de 1 à 8 atomes de carbone, un radical phényle ou méthyl-4 phényle.

On utilise de préférence un chlorure de tétralkylphosphonium.

Le chlorure de tétrabutylphosphonium disponible et particulièrement efficace est plus spécialement recommandé.

On notera que certains composés du palladium, tel $PBu_4PdCl_3$ mentionné précédemment et résultant de la réaction entre des quantités molaires équivalentes de $PBU_4Cl$ et de $PdCl_2$, peuvent constituer à la fois une source de palladium et un moyen d'introduction d'un chlorure d'onium quaternaire au sens indiqué ci-avant.

Il a été constaté que l'effet bénéfique apporté par la présence dans le milieu de carbonylation d'un chlorure d'onium quaternaire correspondant à la définition donnée ci-dessus est sensible à partir d'un rapport molaire cation onium/palladium de 0,5 ; notamment un effet particulièrement intéressant a été constaté lorsque ledit rapport est compris entre 1 et 50, un rapport plus élevé pouvant même être choisi sans préjudice pour la réaction. En effet, le chlorure d'onium quaternaire peut être utilisé en quantité relativement importante et jouer en quelque sorte le rôle supplémentaire de diluant du milieu réactionnel.

La réaction pourra être généralement conduite en phase liquide à une température comprise entre 50 et 150°C, de préférence entre 80 et 130°C, sous une pression d'oxyde de carbone comprise entre 10 et 250 bar (1000 et 25 000 KPa), de préférence entre 15 et 180 bar (1500 et 18 000 KPa).

Des gaz inertes, tels l'azote, l'argon ou le gaz carbonique, peuvent être présents à côté de l'oxyde de carbone.

Bien entendu la réaction peut être conduite en présence de solvants ou diluants exogènes au milieu réactionnel tels les hydrocarbures aromatiques, les esters, les cétones, les nitriles, le diméthylsulfoxyde ou les amides d'acides carboxyliques.

La présence d'eau, même en quantité relativement importante (de l'ordre de 10 moles par mole de substrat) n'est pas nuisible et est susceptible d'avoir, dans certaines conditions, un effet bénéfique sur la sélectivité,

voire sur l'activité.

La présence d'un alcanol inférieur, peut s'avérer souhaitable dans certains cas. En effet, bien qu'un tel alcool ne puisse être considéré comme inerte dans les conditions de réaction, il peut être engagé comme co-réactif pour estérifier l'acide β,γ- formé ce qui peut constituer une alternative pour, par exemple, isoler plus commodément le produit recherché sous sa forme d'ester. Cet ester pourra alors être ensuite hydrolysé de manière en soi connue pour retrouver le produit recherché sous sa forme acide.

Cette variante peut présenter un intérêt plus particulier lorsque l'alcool allylique engagé à la réaction est un un butènediol et lorsque l'alcanol utilisé est le méthanol ou l'éthanol ; le rapport molaire alcanol/diol pourra alors atteindre 10 environ.

Selon une variante avantageuse du procédé selon la présente invention la réaction est conduite dans la N-méthylpyrrolidone.

La concentration de l'alcool allylique n'est pas critique et peut varier dans de larges limites.

En fin de réaction ou du temps de réaction voulu, on récupère l'acide ou le diacide recherché par tout moyen approprié, par exemple par extraction.

Les exemples ci-après illustrent l'invention.

Exemples 1 à 8 ; Essais témoins (a) à (c) :

On réalise une série d'essais selon le mode opératoire décrit par référence à l'exemple 1.

Dans un autoclave en acier inoxydable Hastelloy B2 de 125 cm3, préalablement purgé à l'argon, on introduit :

- 4,4 g (50 mmol) de butène-2 diol-1,4
- 1 matg de palladium sous forme de $PdCl_2$,
- 5 g de $PBu_4Cl$ (17 mmol) et
- 25 cm3 d'acétonitrile

L'autoclave est fermé hermétiquement, placé dans un four agité et raccordé à une alimentation de gaz sous pression. On purge le réacteur à froid avec de l'oxyde de carbone et on chauffe à 100°C ; on régule ensuite la pression à 125 bar. Après 6 heures de réaction (sauf mention contraire) l'autoclave est refroidi et dégazé.

La solution réactionnelle est diluée à 100 cm3 par ajout de solvant.

Une partie aliquote est estérifiée par le méthanol, puis analysée par chromatographie en phase gazeuse.

Tous les essais correspondent à un taux de transformation de 100 % et on observe la formation des divers acides suivants :

- HD : mélange d'acides hexène-3 et hexène-2 dioïques dans lequel l'acide hexène-3 dioïque est majoritaire.
- Ac.$C_5$ : mélange d'acides valérique, méthyl-2 butyrique, pentène-3 oïque, pentène-2 oïque et pentène-4 oïque dans lequel l'acide pentène-3 oïque est majoritaire.
- $C_6$sat. : mélange d'acides éthylsuccinique, méthylglutarique et adipique dans lequel l'acide méthylglutarique est majoritaire.
- PDO : acide pentadiènoïque

dont on indique pour chaque groupe le nombre de moles formées pour 100 moles de butènediol chargées.

Les conditions particulières ainsi que les résultats obtenus figurent dans le tableau I ci-après

TABLEAU I

| Ex n° | Additif - Nature | Additif - mmol | Solvant | t(h) | Résultats (%) - HD | Ac.C5 | sat. C6 | PDO |
|---|---|---|---|---|---|---|---|---|
| a | - | 0 | $CH_3CN$ | | <2 | 60 | 16 | 1 |
| b | $PPh_3$ | 2 | " | | 2 | 0 | 0 | 15 |
| 1 | $PBu_4Cl$ | 17 | " | 3 | 37 | 28 | 9 | 0,5 |
| 2 | $PMe_4Cl$ | " | " | | 19 | 21 | 6 | ND |
| 3 | $PPh_4Cl$ | " | " | | 39 | 30 | 12 | " |
| 4 | $NBu_4Cl$ | " | " | | 30 | 30 | 14 | " |
| c | $PBu_4Br$ | " | " | | 4 | 48 | 15 | " |
| 5 * | $PBu_4Cl$ | 17 | NMP | 1 | 80 | 4 | 3 | 0 |
| 6 * | " | 34 | " | 2 | 85 | 4 | 1 | 0,5 |
| 7 * | " | 68 | " | 1,5 | 92 | 3 | 1 | 0 |
| 8 | " | 102 | Néant | 1,5 | 85 | 3 | 0,5 | 0 |

t(h) : durée d'absorption si elle est inférieure à 6 heures

NMP : N-méthylpyrrolidone

(*) : ($PBu_4Cl$ + NMP) = 30 ml

ND : non déterminé

Exemples 9 A 11 :

Dans l'autoclave et selon le mode opératoire décrits ci-avant pour l'exemple 1, on réalise une seconde série d'essais en remplaçant une partie du butène-2 diol-1,4 par un volume équivalent de solvant, en maintenant constant et égal à 30 cm3 le volume total (solvant + butène-diol). Les conditions particulières ainsi que les résultats obtenus figurent dans le tableau II ci-après.

TABLEAU II

| Ex N° | Butènediol mmol | Solvant | t(h) | Résultats (%) HD | Ac.$C_5$ | C sat.$_6$ | PDO |
|---|---|---|---|---|---|---|---|
| 1 | 50 | $CH_3CN$ | 3 | 37 | 28 | 9 | 0,5 |
| 9 | 25 | " | | 53 | 18 | 13 | 0,5 |
| 5 | 50 | NMP | 1 | 80 | 4 | 3 | 0 |
| 10 | 25 | " | 1 | 85 | 4 | 2 | 0 |
| 11 | 100 | " | | 75 | 2 | 1 | 2,5 |

t(h) : durée d'absorption si elle est inférieure à 6 heures

NMP : N-méthylpyrrolidone

Exemple 12 :

On reproduit l'exemple 1 ci-avant en remplaçant le chlorure de palladium par une quantité équivalente de palladium sous forme de $Pd(OAc)_2$.

En 6 heures de réaction on a obtenu :

- HD : 24 %

Exemple 13 :

On reproduit l'exemple 1 ci-avant en remplaçant le chlorure de palladium par une quantité équivalente de palladium sous forme de $Pd(dba)_2$.

En 6 heures de réaction on a obtenu :

- HD : 32 %

Exemple 14 :

On reproduit l'exemple 1 ci-avant en utilisant un volume équivalent de diméthylsulfoxyde comme solvant.

En 6 heures de réaction on obtient :

- HD : 70 %

Exemple 15 :

On reproduit l'exemple 1 ci-avant en utilisant un volume équivalent de diméthylformamide comme solvant.

En 6 heures de réaction on obtient :

- HD : 50 %

Exemple 16 :

On reproduit l'exemple 5 ci-avant en remplaçant le chlorure de palladium par une quantité équivalente de palladium sous forme de $Pd(OAc)_2$.

En 6 heures de réaction on obtient :

- HD : 45 %

Exemple 17 :

On reproduit l'exemple 5 ci-avant en remplaçant le chlorure de palladium par une quantité équivalente de palladium sous forme de $Pd(dba)_2$.

En 21 heures de réaction on obtient :

- HD : 85 %

Exemple 18 :

On reproduit l'exemple 5 en utilisant 0,5 mat.g de palladium (sous forme de $PdCl_2$).

En 1h 30 mn l'absorbtion est terminée et on obtient sensiblement les mêmes résultats;

- HD : 75 %

Exemple 19 :

On reproduit l'exemple 5 en utilisant 0,12 mat.g de palladium (sous forme de $PdCl_2$).

En 12 h l'absorption est terminée et on obtient sensiblement les mêmes résultats;

- HD : 75 %

Exemple 20 :

On reproduit l'exemple 5 ci-avant en utilisant une quantité équivalente de butène-1 diol-3,4.

On obtient sensiblement les mêmes résultats.

- HD : 80 %

Exemple 21 :

On reproduit l'exemple 5 ci-avant en remplaçant le chlorure de palladium par une quantité équivalente de palladium déposé sur charbon (3 % pds de Pd sur C).

En 1 heure l'absorption est terminée et on obtient :

- HD : 44 %

Exemple 22 :

On reproduit l'exemple 5 ci-avant en remplaçant le chlorure de palladium par une quantité équivalente de $PBu_4PdCl_3$ et en remplaçant $PBu_4Cl$ par un volume équivalent de N-méthyl-pyrrolidone.

En 15 mn l'absorption est terminée et on obtient :

- HD : 45 %

Exemples 23 à 27 :

On réalise une série d'essais analogues à l'exemple 5 ci-avant en remplaçant la N-méthylpyrrolidone (NMP) par le même volume d'un mélange (NMP + eau) ou (NMP + méthanol).

Les conditions particulières et les résultats obtenus sont rassemblés dans le Tableau (III) ci-après :

TABLEAU III

| Ex. n° | Eau (mmol) | méthanol (mmol) | t | HD % |
|---|---|---|---|---|
| 23 | 50 | - | 40 mn | 74,5 |
| 24 | 100 | - | 6 h | 85 |
| 25 | 200 | - | 1 h | 78 |
| 26 | - | 100 | 1 h | 78 |
| 27 | - | 200 | 50 mn | 70 |

t : durée d'absorption.

Exemples 28 à 38 :

On réalise une série d'essais sur une charge analogue à celle indiquée pour l'exemple 1 en utilisant comme solvant soit l'acétonitrile (exemples 28 à 32) soit la N-méthylpyrrolidone (NMP) exemples 33 à 38 en modifiant la température de réaction (T°C) ou la pression de l'oxyde de carbone mesurée en température {P(CO)}.

Les conditions particulières et les résultats obtenus figurent dans le tableau IV ci-après.

TABLEAU IV

| | | | | Résultats (%) | | | |
|---|---|---|---|---|---|---|---|
| Ex n° | P(CO)bar | T°C | t(h) | HD | Ac.C 5 | C sat. 6 | PDO |
| 28 | 60 | 100 | 4,5 | 20 | 28 | 12 | 0 |
| 29 | 120 | " | 3 | 41 | 18 | 15 | 0 |
| 30 | 180 | " | 3 | 44 | 13 | 2 | 0 |
| 31 | 120 | 130 | 3 | 18 | 30 | 13 | 1 |
| 32 | " | 70 | | 43 | 5 | 2 | 2 |
| 33 | 15 | 100 | (*) | 15 | 46 | 2 | 2,5 |
| 34 | 30 | " | | 56 | 17 | 3 | 1,5 |
| 35 | 60 | " | 2 | 80 | 6 | 1 | 2 |
| 5 | 120 | " | 1 | 80 | 4 | 3 | 0 |
| 36 | 180 | " | 2 | 82 | 2 | 0 | 3 |
| 37 | 120 | 130 | 0,66 | 55 | 14 | 2 | 2 |
| 38 | ." | 70 | | 70 | 1 | 0 | 5 |

**t(h) : durée d'absorption si elle est inférieure à 6 heures**

**NMP : N-méthylpyrrolidone**

**(*) : durée d'absorbtion = 18 heures**

Exemple 39 :

Dans un appareillage et selon un mode opératoire analogue à celui décrit précédemment on réalise un essai sur une charge constituée par :

- 50 mmol d'alcool allylique
- 37,5 cm3 d'éthylbenzène
- 0,25 mAtg de palladium introduit sous forme de $PdCl_2$
- 2,5 mmol de $PBu_4Cl$

La température est de 80°C et la pression régulée à 200 bar. Après 6 heures de réaction l'autoclave est refroidi et dégazé.

L'analyse du mélange brut par chromatographie en phase gazeuse montre que le taux de transformation de l'alcool allylique est de 100 %, et que le mélange renferme du vinylacétate d'allyle (RR = 60 % ; RR étant défini par le rapport du double du nombre de moles de vinylacétate détecté au nombre de moles d'alcool allylique chargé) et de l'acide butène-3 oïque dont la quantité estimée comme suit est de l'ordre de 30 % par rapport à l'alcool allylique chargé.

L'estimation est faite par différence entre, d'une part la quantité totale d'ester méthylique dosé par chromatographie en phase gazeuse après estérification et, d'autre part la quantité d'ester méthylique résultant de la dégradation du vinylacétate d'allyle, l'analyse du mélange, après estérification, par chromatographie en phase gazeuse ayant conduit aux résultats suivants :

RR (vinylacétate d'allyle) = 20 %

RR (butène-3 oate de méthyle) = 50 %
(RR : rendement molaire par rapport au substrat engagé)

Essai témoin (d) :

On reproduit l'exemple 39 ci-avant en introduisant une quantité équivalente de palladium sous forme du complexe

$PdCl_2 (PPh_3)_2$

et en remplaçant $PBu_4Cl$ par une quantité équivalente de $SnCl_2.2H_2O$

Les résultats obtenus toutes conditions égales par ailleurs sont les suivants :

Analyse du mélange brut :

Taux de transformation de l'alcool allylique : 15 %
RR (vinylacétate d'allyle) : 16 %

Analyse du mélange après estérification :

RR (vinylacétate d'allyle) : 8 %
RR (butène-3 oate de méthyle) : 6 %

La comparaison des résultats obtenus respectivement dans l'exemple 39 (selon l'invention) et dans l'essai témoin (d) met en évidence le fait qu'avec le procédé selon l'invention on accède à l'acide β,γ-insaturé et ce, avec une efficacité de carbonylation améliorée. Exemple 40 :

Dans un appareillage et selon un mode opératoire analogue à celui décrit précédemment on réalise une essai sur une charge constituée par :

40 mmol d'alcool allylique
25 cm3 de N-méthylpyrrolidone
1 mmol de $PdCl_2$
17 mmol de $PBU_4Cl$

La température est de 100°C et la pression régulée à 120 bar. Après 30 mn de réaction (durée d'absorption) les résultats obtenus sont les suivants.

Analyse du mélange brut :

Taux de transformation de l'acool allylique : 100 %
RR (vinylacétate d'allyle) : 0 %

Analyse du mélange après estérification :

RR (vinylacétate d'allyle) : 0 %
RR (butène-3 oate de méthyle) : 62 %

Cet exemple montre que l'on forme sélectivement l'acide β,γ-insaturé et ce, avec une efficacité appréciable de carbonylation.

Exemple 41 :

On reproduit l'exemple 40 ci-avant en ajoutant à la charge 100 mmol d'eau. Après 10 minutes de réaction (durée d'absorption), les résultats obtenus sont les suivants :

Analyse du mélange brut :

Taux de transformation de l'alcool allylique : 100 %
RR (vinylacétate d'allyle) : 2 %

Analyse du mélange après estérification :

RR (vinylacétate d'allyle) : 0 %

RR (butène-3 oate de méthyle) : 100 %

Essai témoin (e) :

On reproduit l'exemple 40 ci-avant en remplaçant le chlorure de palladium par une quantité équivalente du complexe $PdCl_2(PPh_3)_2$ et $PBU_4Cl$ par 10 mmol de $SnCl_2$, $2H_2O$. Après 4 heures de réactions (durée d'absorption), les résultats obtenus sont les suivants :

Analyse du mélange brut :

Taux de transformation de l'alcool allylique : 10 %
RR (vinylacétate d'allyle) : 2 %

Analyse du mélange après estérification :

RR (vinylacétate d'allyle) : 0 %
RR (butène-3 oate de méthyle) : 5 %

Exemples 42 à 46 :

Dans l'autoclave et selon le mode opératoire décrits précédemment on réalise une série d'essais au départ de divers alcools allyliques dans les conditions communes suivantes :

La charge est constituée par :
- 50 mmol d'alcool dont la nature est précisée dans le tableau V ci-après :
- 1 mat.g de palladium sous forme de $PdCl_2$
- 17 mmol de $PBU_4Cl$
- 25 cm3 de N-méthylpyrrolidone

La température de réaction est de 100°C et la pression de l'oxyde de carbone mesurée en température est de 120 bar.

En fin de réaction le mélange réactionnel brut est analysé par chromatographie en phase gazeuse, puis estérifié par le méthanol ; la quantité d'acide β,γ-insaturé est déterminée par dosage en chromatographie en phase gazeuse des esters méthyliques correspondants

TT : représente le taux de transformation de l'alcool considéré.
RR : représente le nombre de moles de l'acide β,γ-insaturé formé pour 100 moles d'alcool chargé.

TABLEAU V

| Ex. n° | Nature de l'alcool | t(h) | TT | RR |
|---|---|---|---|---|
| 42 | Méthyl-2 propène-1 ol-3 | 3,5 | 90 | 50 |
| 43 | Butène-2 ol-1 | 3,5 | 100 | 92 |
| 44 | Cyclohexène-2 ol-1 | 3,5 | 100 | 82 |
| 45 | Phényl-3 propène-2 ol-1 | 1 | 92 | 85 |
| 46 | Méthyl-3 butène-1 ol-3 | 0,5 | 100 | 100 |

**Revendications**

1. Procédé de préparation d'acides β,γ-insaturés par mise en contact d'un alcool allylique, de l'oxyde de carbone et d'un catalyseur à base de palladium, à température élevée et sous une pression supérieure à la pression atmosphérique caractérisé en ce qu'on opère également en présence d'au moins un chlorure d'onium quaternaire d'un élément du groupe VB choisi parmi l'azote et le phosphore, ledit élément étant tétracoordonné à des atomes de carbone, l'azote pouvant être coordonné à deux atomes de phosphore pentavalents.

2. Procédé selon la revendication 1, caractérisé en ce que l'alcool allylique répond à la formule (I) :

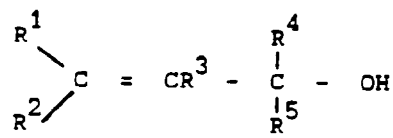

dans laquelle

. $R^1$ à $R^5$ identiques ou différents représentent :
  - un atome d'hydrogène,
  - un radical alkyle, aryle ou aralkyle comportant au maximum 20 atomes de carbone,
  - un radical alcényle exempt d'insaturation terminale et renfermant au maximum 15 atomes de carbone,

. l'un quelconque des radicaux $R^1$ à $R^5$ peut en outre représenter un radical hydroxyméthyle ($-CH_2OH$),
. $R^1$(ou $R^2$) et $R^4$(ou $R^5$) peuvent en outre former ensemble un radical unique divalent alkylène comportant de 2 à 5 atomes de carbone.

3. Procédé selon la revendication 1 ou 2 caractérisé en ce que le chlorure d'onium quaternaire présente un cation onium quaternaire répondant à l'une des formules II à V ci-après :

II)

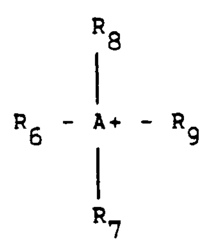

III)

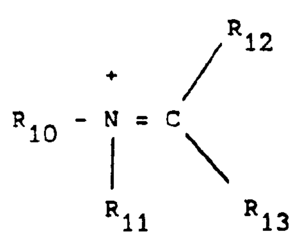

IV)

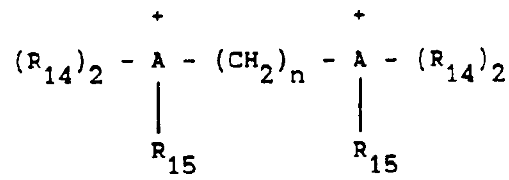

v) $(R_{16})_3 - P = N^+ = P - (R_{16})_3$

dans lesquelles :

- A représente de l'azote ou du phosphore
- $R_6$, $R_7$,$R_8$, $R_9$ sont identiques ou différents et répresentent :
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préference de 4 à 8 atomes de carbone,
  . un radical aryle contenant de 6 à 10 atomes de carbone, éventuellement substitué par un ou des radicaux alkyles contenant de 1 à 4 atomes de carbone, alcoxy, alcoxycarbonyle ou halogéno ;
  . deux desdits radicaux $R_6$ à $R_9$ pouvant former ensemble un radical alkylène, alcénylène ou alcadiènylène, linéaire ou ramifié et contenant de 3 à 6 atomes de carbone ;
- $R_{10}$, $R_{11}$, $R_{12}$, $R_{13}$ sont identiques ou différents et représentent :
  . un radical alkyle linéaire ou ramifié contenant de 1 à 4 atomes de carbone ;
  . les radicaux $R_{12}$ et $R_{13}$ pouvant former ensemble un radical alkylène, contenant de 3 à 6 atomes de carbone ;
  . les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec N un hétérocycle azoté ;
- $R_{14}$ représente un radical alkyle linéaire ou ramifié contenant de 1 à 4 atomes de carbone ou un radical phényle ;
- $R_{15}$ représente :
  . un radical alkyle linéaire ou ramifié contenant de 1 à 4 atomes de carbone, semblable ou différent de $R_{14}$ ;
  . un radical alcényle linéaire ou ramifié contenant de 2 à 12 atomes de carbone, de préférence de 4 à 8 atomes de carbone ;
- n représente un nombre entier supérieur ou égal à 1 et inférieur ou égal à 10 et de préférence inférieur ou égal à 6 ;
- $R_{16}$ représente un radical aryle contenant de 6 à 10 atomes de carbone, éventuellement substitué par un ou des groupes alkyle contenant de 1 à 4 atomes de carbone, alcoxy, alcoxycarbonyle ou halogéno.

**4.** Procédé selon l'une quelconque des revendications précédentes caractérisé en ce que le cation onium quaternaire répond à la formule (II) donnée dans la revendication 3, dans laquelle :

- A représente du phosphore et,
- $R_6$, $R_7$, $R_8$ et $R_9$ sont identiques ou différents et représentent un radical alkyle linéaire ou ramifié contenant de 1 à 8 atomes de carbone, un radical phényle ou méthyl-4 phényle.

**5.** Procédé selon l'une quelconque des revendications précédentes, caractérisé en ce que le chlorure d'onium quaternaire est le chlorure de tétrabutylphosphonium.

**6.** Procédé selon l'une quelconque des revendications précédentes, caractérisé en ce que le rapport molaire du cation onium au palladium est supérieur ou égal à 1.

**7.** Procédé selon l'une quelconque des revendications précédentes, caractérisé en ce que la concentration du palladium dans le milieu réactionnel est comprise entre $10^{-3}$ et 1 mol/l.

8. Procédé selon l'une quelconque des revendications précédentes, caractérisé en ce que la température de réaction est comprise entre 50 et 150°C et, de préférence, entre 80 et 130°C.

9. Procédé selon l'une quelconque des revendications précédentes, caractérisé en ce que la pression est comprise entre 10 et 250 bar (1000 et 25000 KPa) et, de préférence, entre 15 et 180 bar (1500 et 18 000 KPa).

10. Procédé selon l'une quelconque des revendications précédentes, caractérisé en ce que la réaction est conduite en présence d'un solvant ou diluant organique.

11. Procédé selon la revendication 10, caractérisé en ce que le solvant est la N-méthylpyrrolidone.

12. Procédé selon la revendication 10, caractérisé en ce que le solvant est le chlorure d'onium quaternaire.

13. Procédé selon l'une quelconque des revendications précédentes caractérisé en ce que l'alcool allylique est choisi parmi le butène-2 diol-1,4, le butène-1 diol-3,4 et leurs mélanges.

14. Procédé selon la revendication 13, caractérisé en ce que la réaction est conduite en présence d'un alcanol inférieur.

15. Procédé selon la revendication 13, caractérisé en ce que la réaction est conduite en présence d'eau.

## Claims

1. Process for preparing β,γ-unsaturated acids by bringing an allyl alcohol, carbon monoxide and a palladium-based catalyst into contact at high temperature and under a pressure above atmospheric pressure, characterised in that the reaction is also performed in the presence of at least one quaternary onium chloride of a group VB element selected from nitrogen and phosphorus, the said element being four-coordinated to carbon atoms, it being possible for the nitrogen to be coordinated to two pentavalent phosphorus atoms.

2. Process according to claim 1, characterised in that the allyl alcohol corresponds to the formula (I):

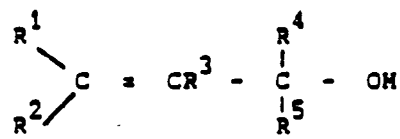

in which:

. $R^1$ to $R^5$, which may be identical or different, represent:
  - a hydrogen atom,
  - an alkyl, aryl or aralkyl radical containing at most 20 carbon atoms,
  - an alkenyl radical free from terminal unsaturation and containing at most 15 carbon atoms,

. any one of the radicals $R^1$ to $R^5$ can, in addition, represent a hydroxymethyl radical ($-CH_2OH$),

. $R^1$ (or $R^2$) and $R^4$ (or $R^5$) together can, in addition, form a single divalent alkylene radical containing from 2 to 5 carbon atoms.

3. Process according to claim 1 or 2, characterised in that the quaternary onium chloride possesses a quaternary onium cation corresponding to one of the formulae II to V below:

II)

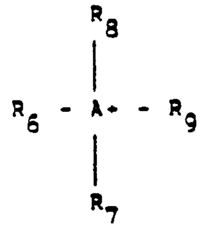

III)

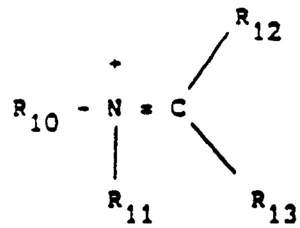

IV)

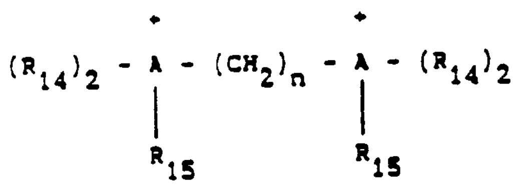

v) $(R_{16})_3 - P = N^+ = P - (R_{16})_3$

in which:

- A represents nitrogen or phosphorus;
- $R_6$, $R_7$, $R_8$ and $R_9$ are identical or different and represent:
  . a linear or branched alkyl radical containing from 1 to 16 carbon atoms, optionally substituted with a phenyl, hydroxy, halo, nitro, alkoxy or alkoxycarbonyl group;
  . a linear or branched alkenyl radical containing from 2 to 12 carbon atoms, and preferably from 4 to 8 carbon atoms;
  . an aryl radical containing from 6 to 10 carbon atoms, optionally substituted with one or more alkyl radicals containing from 1 to 4 carbon atoms, or alkoxy, alkoxycarbonyl or halo radicals;
  . it being possible for two of the said radicals $R_6$ to $R_9$ together to form a linear or branched alkylene, alkenylene or alkadienylene radical containing from 3 to 6 carbon atoms;
- $R_{10}$, $R_{11}$, $R_{12}$ and $R_{13}$ are identical or different and represent:
  . a linear or branched alkyl radical containing from 1 to 4 carbon atoms;
  . it being possible for the radicals $R_{12}$ and $R_{13}$ together to form an alkylene radical containing from 3 to 6 carbon atoms;
  . it being possible for the radicals $R_{11}$ and $R_{12}$ or $R_{11}$ and $R_{13}$ together to form an alkylene, alkenylene or alkadienylene radical containing 4 carbon atoms and constituting with N a nitrogenous heterocycle;
- $R_{14}$ represents a linear or branched alkyl radical containing from 1 to 4 carbon atoms or a phenyl radical;
- $R_{15}$ represents:
  . a linear or branched alkyl radical containing from 1 to 4 carbon atoms, similar to or different from $R_{14}$;
  . a linear or branched alkenyl radical containing from 2 to 12 carbon atoms, and preferably from 4 to 8 carbon atoms;
- n represents an integer not less than 1 and not more than 10, and preferably not more than 6;
- $R_{16}$ represents an aryl radical containing from 6 to 10 carbon atoms, optionally substituted with one or more alkyl groups containing from 1 to 4 carbon atoms, or alkoxy, alkoxycarbonyl or halo groups.

**4.** Process according to any one of the preceding claims, characterised in that the quaternary onium cation

corresponds to the formula II given in claim 3, in which:

- A represents phosphorus, and
- $R_6$, $R_7$, $R_8$ and $R_9$ are identical or different and represent a linear or branched alkyl radical containing from 1 to 8 carbon atoms, or a phenyl or 4-methylphenyl radical.

**5.** Process according to any one of the preceding claims, characterised in that the quaternary onium chloride is tetrabutylphosphonium chloride.

**6.** Process according to any one of the preceding claims, characterised in that the mole ratio of the onium cation to palladium is not less than 1.

**7.** Process according to any one of the preceding claims, characterised in that the concentration of palladium in the reaction medium is between $10^{-3}$ and 1 mol/1.

**8.** Process according to any one of the preceding claims, characterised in that the reaction temperature is between 50 and 150°C, and preferably between 80 and 130°C.

**9.** Process according to any one of the preceding claims, characterised in that the pressure is between 10 and 250 bars (1,000 and 25,000 kPa), and preferably between 15 and 180 bars (1,500 and 18,000 kPa).

**10.** Process according to any one of the preceding claims, characterised in that the reaction is performed in the presence of an organic diluent or solvent.

**11.** Process according to claim 10, characterised in that the solvent is N-methylpyrrolidone.

**12.** Process according to claim 10, characterised in that the solvent is the quaternary onium chloride.

**13.** Process according to any one of the preceding claims, characterised in that the allyl alcohol is selected from 2-butene-1,4-diol, 1-butene-3,4-diol and mixtures thereof.

**14.** Process according to claim 13, characterised in that the reaction is performed in the presence of a lower alkanol.

**15.** Process according to claim 13, characterised in that the reaction is performed in the presence of water.

## Patentansprüche

**1.** Verfahren zur Herstellung β,γ-ungesättigter Säuren, indem man einen allylischen Alkohol, Kohlenoxid und einen Katalysator auf Palladium-Basis bei erhöhter Temperatur und unter höherem Druck als Atmosphärendruck in Kontakt bringt, dadurch gekennzeichnet, daβ man ebenfalls in Gegenwart von mindestens einem quartären Oniumchlorid eines Elements der Gruppe VB, ausgewählt aus Stickstoff und Phosphor, arbeitet, wobei das besagte Element vierfach mit Kohlenstoffatomen koordiniert ist, wobei der Stickstoff mit zwei fünfwertigen Phosphoratomen koordiniert sein kann.

**2.** Verfahren nach Anspruch 1, dadurch gekennzeichnet, daβ der allylische Alkohol der Formel (I) entspricht:

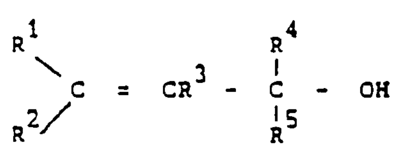

in der

$R^1$ bis $R^5$, die gleich oder verschieden sein können,

- ein Wasserstoffatom,
- einen Alkyl-, Aryl- oder Aralkylrest, der maximal 20 Kohlenstoffatome umfaβt,
- einen Alkenylrest frei von terminaler Nicht-Sättigung und maximal 15 Kohlenstoffatome einschlieβend,

darstellen,
irgendeiner der Reste $R^1$ bis $R^5$ darüber hinaus einen Hydroxymethylrest ($-CH_2OH$) darstellen kann,
$R^1$ (oder $R^2$) und $R^4$ (oder $R^5$) darüber hinaus zusammen einen einzigen zweiwertigen Alkylenrest, der zwei bis fünf Kohlenstoffatome umfaßt, bilden können.

3. Verfahren nach Anspruch 1 oder 2, dadurch gekennzeichnet, daß das quartäre Oniumchlorid ein quartäres Onium-Kation aufweist, das einer der nachstehenden Formeln II bis V entspricht:

II)

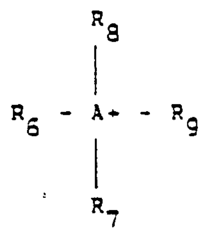

III)

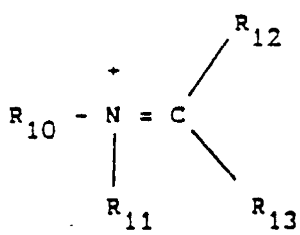

IV)

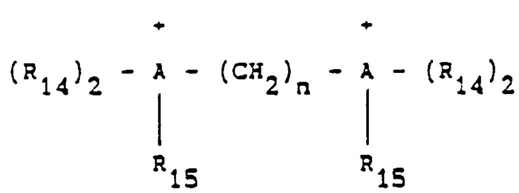

v) $(R_{16})_3 - P = N^+ = P - (R_{16})_3$

in denen:

- A Stickstoff oder Phosphor darstellt,
- $R_6$, $R_7$, $R_8$, $R_9$ gleich oder verschieden sind und darstellen:
  einen linearen oder verzweigten Alkylrest mit 1 bis 16 Kohlenstoffatomen, der gegebenenfalls durch eine Phenyl-, Hydroxy-, Halogen-, Nitro-, Alkoxy- oder Alkoxycarbonyl-Gruppe substituiert ist; einen linearen oder verzweigten Alkenylrest mit 2 bis 12 Kohlenstoffatomen, vorzugsweise 4 bis 8 Kohlenstoffatomen;
  einen Arylrest mit 6 bis 10 Kohlenstoffatomen, der gegebenenfalls durch einen oder mehrere Alkylreste mit 1 bis 4 Kohlenstoffatomen, Alkoxy-, Alkoxycarbonyl- oder Halogenreste substituiert ist; wobei zwei der besagten Reste $R_6$ bis $R_9$ zusammen einen Alkylen-, Alkenylen- oder Alkadienylenrest, geradkettig oder verzweigt und 3 bis 6 Kohlenstoffatome enthaltend, bilden können;
- $R_{10}$, $R_{11}$, $R_{12}$, $R_{13}$ gleich oder verschieden sind und darstellen:
  einen linearen oder verzweigten Alkylrest mit 1 bis 4 Kohlenstoffatomen;
  wobei
  die Reste $R_{12}$ und $R_{13}$ zusammen einen 3 bis 6 Kohlenstoffatome enthaltenden Alkylenrest bilden

können;
die Reste $R_{11}$ und $R_{12}$ oder $R_{11}$ und $R_{13}$ zusammen einen Alkylen-, Alkenylen- oder Alkadienylenrest bilden können, der 4 Kohlenstoffatome enthält und zusammen mit N einen Stickstoffheterocyclus bildet;

- $R_{14}$ einen linearen oder verzweigten Alkylrest mit 1 bis 4 Kohlenstoffatomen oder einen Phenylrest darstellt;
- $R_{15}$ darstellt:
  einen linearen oder verzweigten Alkylrest mit 1 bis 4 Kohlenstoffatomen, der gleich oder verschieden von $R_{14}$ ist;
  einen linearen oder verzweigten Alkenylrest mit 2 bis 12 Kohlenstoffatomen, vorzugsweise 4 bis 8 Kohlenstoffatomen;
- n eine ganze Zahl größer oder gleich 1 und kleiner oder gleich 10 und vorzugsweise kleiner oder gleich 6 darstellt;
- $R_{16}$ einen Arylrest mit 6 bis 10 Kohlenstoffatomen, welcher gegebenenfalls durch 1 oder mehrere Alkylgruppen mit 1 bis 4 Kohlenstoffatomen, Alkoxy-, Alkoxycarbonyl- oder Halogengruppen substituiert ist, darstellt.

**4.** Verfahren nach irgendeinem der vorangehenden Ansprüche, dadurch gekennzeichnet, daß das quartäre Onium-Kation der im Anspruch 3 gegebenen Formel (II) entspricht, in der:
- A Phosphor darstellt und
- $R_6$, $R_7$, $R_8$ und $R_9$ gleich oder verschieden sind und einen linearen oder verzweigten Alkylrest mit 1 bis 8 Kohlenstoffatomen, einen Phenyl- oder 4-Methylphenylrest darstellen.

**5.** Verfahren nach irgendeinem der vorangehenden Ansprüche, dadurch gekennzeichnet, daß das quartäre Oniumchlorid Tetrabutylphosphoniumchlorid ist.

**6.** Verfahren nach irgendeinem der vorangehenden Ansprüche, dadurch gekennzeichnet, daß das Molverhältnis des Onium- Kations zu Palladium größer oder gleich 1 ist.

**7.** Verfahren nach irgendeinem der vorangehenden Ansprüche, dadurch gekennzeichnet, daß die Konzentration des Palladiums im Reaktionsmedium zwischen $10^{-3}$ und 1 mol/l eingeschlossen liegt.

**8.** Verfahren nach irgendeinem der vorangehenden Ansprüche, dadurch gekennzeichnet, daß die Reaktionstemperatur zwischen 50 und 150° C und vorzugsweise zwischen 80 und 130°C eingeschlossen ist.

**9.** Verfahren nach irgendeinem der vorangehenden Ansprüche, dadurch gekennzeichnet, daß der Druck zwischen 10 und 250 bar (1000 und 25000 kPa) und vorzugsweise zwischen 15 und 180 bar (1500 und 18000 kPa) eingeschlossen ist.

**10.** Verfahren nach irgendeinem der vorangehenden Ansprüche, dadurch gekennzeichnet, daß die Reaktion in Gegenwart eines organischen Lösungsmittels oder Verdünnungsmittels durchgeführt wird.

**11.** Verfahren nach Anspruch 10, dadurch gekennzeichnet, daß das Lösungsmittel N-Methylpyrrolidon ist.

**12.** Verfahren nach Anspruch 10, dadurch gekennzeichnet, daß das Lösungsmittel das quartäre Oniumchlorid ist.

**13.** Verfahren nach irgendeinem der vorangehenden Ansprüche, dadurch gekennzeichnet, daß der allylische Alkohol aus Buten-2-diol-1,4, Buten-1-diol-3,4 und deren Mischungen ausgewählt ist.

**14.** Verfahren nach Anspruch 13, dadurch gekennzeichnet, daß die Reaktion in Gegenwart eines niederen Alkanols durchgeführt wird.

**15.** Verfahren nach Anspruch 13, dadurch gekennzeichnet, daß die Reaktion in Gegenwart von Wasser durchgeführt wird.